# EUROPEAN PATENT APPLICATION

(11) **EP 3 427 825 A1**
(43) Date of publication of application: **16.01.2019**
(21) Application number: 18165758.6
(22) Date of filing: 09.05.2012
(51) Int. Cl.: B01J 31/22, B01J 31/20, B01J 31/12, C07C 6/04

(54) **HYBRID CATALYST FOR OLEFIN METATHESIS**

(30) Priority: 10.05.2011 US 201113104643
(62) Divisional of application: 12722604.1
(71) Applicant: Saudi Arabian Oil Company, Dhahran 31311 (SA)
(72) Inventor: Wang, Yuguo, Dhahran 31311 (SA); Ercan, Cemal, 31311 Dhahran (SA)
(74) Representative: Stafford, Jonathan Alan Lewis

(57) **Abstract**

A method of preparing a hybrid olefin metathesis catalyst is provided. The method comprises the steps of:
- contacting a molecular olefin metathesis catalyst, optionally dissolved in an organic solvent, with a silica support that includes a hydroxyl ligand capable of participating in a ligand exchange reaction;
- appending the olefin metathesis catalyst to the silica support via the ligand exchange reaction to form a hybrid olefin metathesis catalyst; and
- recovering the hybrid olefin metathesis catalyst.

Preferably the molecular olefin metathesis catalyst is the Grubbs-I and/or Grubbs-II catalyst.

## Description

### Field of the Invention

This invention relates to a catalyst and method of preparing a catalyst for olefin metathesis reactions.

### Background of the Invention

Catalytic olefin metathesis is a popular and useful chemical reaction that is able to transform simple and cheap organic molecules into complex and valuable molecules. Typically, for olefin metathesis reactions, transition-metal catalyst compounds are used, such as metal carbenes. In olefin metathesis, two olefin molecules exchange the groups around the double bonds in the presence of a catalyst. The olefins can be of different molecules by structure and composition, or two identical molecules. In general, reaction temperatures for olefin metathesis reactions can be as low as at room temperature or can be at temperatures up to about 500°C or greater, depending on the type of starting materials, the catalyst used, and the media in which the reaction is carried out.

Olefin metathesis reactions have been responsible for opening up new synthetic routes to industrial important petrochemicals, oleochemicals, polymers, and specialty chemicals. Two well known olefin metathesis processes previously or currently in use in the petrochemical industry include the Olefins Conversion Technology (OCT) process (originally known as the Phillips triolefin process) and the Shell Higher Olefins Process (SHOP). In many of the petrochemical olefin metathesis processes that are currently being practiced, the processes utilize heterogeneous catalysts selected from tungsten oxides supported on silica, molybdenum supported on alumina or rhenium oxide supported on alumina. The reaction operating temperatures for each catalyst varies greatly as the rhenium oxide/alumina catalysts typically operate at reaction temperatures that are at or near room temperature, while molybdenum oxide/alumina catalysts typically operate at reaction temperatures between about 100°C to 200°C, and the tungsten oxide/silica catalysts typically operate at reaction temperatures of between about 200°C to 500°C, or greater.

Currently, the most commonly used olefin catalysts in the petrochemical industry are heterogeneous catalysts based upon tungsten oxide/silica. These catalysts are generally preferred because they are less susceptible to poisoning by trace quantities of catalyst poisons in the feed stream (for example, water, air, acetone, carbon monoxide, hydrogen, methanol, and the like) than typically experienced with the lower temperature alumina based rhenium and molybdenum-based catalysts. The tungsten oxide/silica catalysts, however, suffer in that the higher operating temperatures favors both coking and deactivation of the catalyst, which in turn requires frequent catalyst regeneration. Another disadvantage of high operating temperatures is an increased amount of isomerization of the molecules in the feed or products, which in turn can cause faster deactivation of the catalyst and lower selectivity. These effects are believed to be due, in part, to increased acidity of both Bronsted and Lewis acidic sites on the surface of the silica support at the higher temperature.

One commercially available tungsten oxide/silica heterogeneous metathesis catalyst currently being used in the industry operates at temperatures above about 260°C. At these elevated temperatures, coking and rapid deactivation the catalyst occurs, thus requiring more frequent regeneration. Additionally, the high operating temperatures result in the production of increased amounts of isomerization products, while at the same time reducing the selectivity of the reaction to certain desired products. In contrast, olefin reactions utilizing homogeneous catalysts typically require a reaction temperature of only about 50°C or lower, have a higher selectivity to desired products, and provides better overall reaction control. The homogeneous catalysts, however, are not without their disadvantages, namely the substantial difficulties in separating the catalyst from the reactants and products of the reaction.

There are numerous homogeneous catalysts that have been reported thus far for use in olefin metathesis reactions. These catalysts include ruthenium-based first and second generation Grubbs catalysts; molybdenum-based Schrock catalysts; molybdenum hexacarbonyl (Mo(CO)₆); rhenium pentacarbonyl (Re(CO)₅); ruthenium tetracarbonyl (Ru(CO)₄); (methylmethoxycarbene) pentacarbonyl tungsten ((CO)₅W=C(CH₃)(OCH₃)); tungsten hexachloride; methyllithium; tetramethyltin; and molybdenum pentachloride. The Grubbs and Schrock catalysts are particularly well known catalysts that have been used in the synthesis of new molecules, such as epothilones, carbohydrate-containing polymers having biological activity, and for the total synthesis of certain natural product compounds. The Schrock catalysts are known to be air and moisture sensitive, thus severely limiting their usefulness with respect to industrial applications. For metathesis reactions, the homogeneous Schrock catalysts are typically dissolved in ionic liquids, thereby allowing for easier separation of the products from the catalysts. While the Grubbs and Schrock catalysts are known catalysts for olefin metathesis, as homogeneous catalysts, the difficulty in separating these catalysts from the reactants and products severely limits the industrial utility of these catalysts.

As the current heterogeneous catalysts require high reaction temperatures, frequent regeneration, and have low selectivity, and current homogeneous catalysts have limited industrial use due to the difficulty in separation from products and reactants, there exists for a high activity heterogeneous olefin metathesis catalyst that provides improved reaction control, reduced reaction temperature, and higher selectivity to the desired products, as well as ease of separation from reactants and products.

### Summary

The current invention provides a hybrid catalyst for olefin metathesis reactions, specifically the metathesis of ethenes and butenes, and butene self-metathesis.

In one aspect, the present invention provides a hybrid olefin metathesis catalyst. The catalyst includes a homogeneous olefin metathesis catalyst appended to a solid support material. The olefin metathesis catalyst is selected from a homogeneous tungsten-based, molybdenum-based, or ruthenium-based catalyst. The solid support is a high surface area porous support; and the catalyst is appended to the solid support with a ligand.

In another aspect, a method of preparing the a hybrid olefin metathesis catalyst is provided. The method includes the steps of: providing a surface modified solid support material. The solid support material includes a ligand capable of participating in a ligand exchange reaction. The method further includes the step of appending a homogeneous olefin catalyst to a solid support material via ligand exchange reaction, wherein the ligand is selected from halogen or hydroxyl groups. Finally, the method includes the step of recovering the hybrid olefin metathesis catalyst.

In another aspect, a method of preparing the a hybrid olefin metathesis catalyst is provided. The method includes the steps of providing a surface modified high surface area solid support material, the solid support material including hydroxyl groups appended thereto. The method also includes the step of contacting the surface modified solid support material that includes a metal selected from the group consisting of tungsten, molybdenum and ruthenium with a homogeneous olefin metathesis catalyst such that the catalyst binds to the surface of the support material to produce the hybrid catalyst.

In another aspect, a method for the metathesis of butene to produce propene. The method includes the steps of providing an olefin feedstream comprising 1-butene, 2-butene or a mixture thereof to a reaction chamber. Contacting the feedstream with a heterogeneous olefin metathesis catalyst at a temperatures of less than 100°C, wherein the catalyst includes a ruthenium-based, molybdenum-based, or tungsten based olefin metathesis catalyst appended to a solid support. The method further includes the steps of withdrawing a product stream comprising propene; and separating the catalyst from the product stream.

### Detailed Description of the Invention

Although the following detailed description contains many specific details for purposes of illustration, it is understood that one of ordinary skill in the art will appreciate that many examples, variations and alterations to the following catalysts, methods of making the catalysts, and uses thereof are within the scope and spirit of the invention. Accordingly, the exemplary embodiments of the invention described herein are set forth without any loss of generality, and without imposing limitations, relating to the claimed inventions.

Olefin metathesis reactions take place between two molecules that include double bonds. The groups bonded to the carbon atoms of the double bond are exchanged between the molecules to produce two new molecules containing double bonds with the swapped groups. The specific catalyst that is selected for the olefin metathesis reaction generally helps to determine whether a cis- isomer or trans- isomer is formed, as the coordination of the olefin molecules with the catalyst play an important role, as do the steric influences of the substituents on the double bond of the newly formed molecule.

### Hybrid Catalyst Composition

In one aspect, the present invention is directed to hybrid olefin metathesis catalysts having homogeneous catalysts immobilized on high surface area porous heterogeneous support materials for the cross metathesis of ethene and butene and/or the self-metathesis of butenes to produce propylene and other alkenes. These homogeneous olefin metathesis catalysts, particularly the first and second generation Grubbs catalysts immobilized onto the surface of a heterogeneous catalyst support, are herein referred to as hybrid catalysts.

Among the known homogeneous olefin metathesis catalysts suitable for immobilization to the solid support include those typically used in the pharmaceutical and agricultural areas. These include ruthenium based Grubbs' catalysts (first and second generation catalysts); molybdenum based Schrock's catalysts; molybdenum hexacarbonyl (MO(CO)₆); rhenium pentacarbonyl (Ru(CO)₅); ruthenium tetracarbonyl (Ru(CO)₄); (methylmethoxycarbene) pentacarbonyl tungsten ((CO)₅W=C(CH₃)(OCH₃)); tungsten hexachloride; methyllithium; tetramethyltin; molybdenum pentachloride; rhenium pentachloride; and the like.

In certain embodiments, immobilization of homogeneous catalysts, such as the Grubbs and Schrock catalysts, on the surface of a solid support to produce a hybrid catalyst for olefin metathesis maintains the activity of the homogeneous catalyst, while at the same time providing a catalyst that is easily separated from the reactants and products, thereby making it practical to use the hybrid catalysts for industrial applications.

Suitable support materials or "carriers" for the immobilization of the homogeneous catalysts can have the following properties: thermal and chemical resistance, a high surface area, and a relatively high number of centers for chemisorption of the homogeneous catalyst molecule. In certain embodiments, the number of chemisorption centers is between 4.5 and 42.7 centers/nm². In certain embodiments, the number of chemisorption centers is between 5 and 45 chemisorption centers/nm², alternatively between 5 and 20 chemisorption centers/nm², alternatively between 15 and 35 chemisorption centers/nm², or alternatively between 25 and 45 chemisorption centers/nm². In certain embodiments, the substrate materials can be catalytically inactive with respect to olefin metathesis reactions. In certain embodiments, the substrate can be a highly porous material having a surface areas of at least 20 m²/g or greater, alternatively at least 50 m²/g or greater, alternatively at least 100 m²/g or greater, or alternatively at least 200 m²/g or greater, such as silica, alumina, titania, zirconia, hafnia, and niobia. Alternatively, the solid support can be activated carbon. In certain embodiments, the substrate materials can include alumina or silicon carbide ceramic forms having a relatively low surface area, such as between 2 and 10 m²/g. In certain embodiments, the support material can be surface modified polymer. Exemplary polymers can include ion-exchange cross-linked polystyrene, which in certain embodiments can include surface groups, such as sulfonic or sulfonate groups. In certain embodiments, the polymer surface can include hydroxyl functional groups. The polymer can be of any useful shape, size or form, such as pellets, spheres, or other shapes suitable for the selected reactor type. In certain embodiments, a functionalized polymer can be used to coat the surface of a substrate, optionally porous, for subsequent attachment of the homogeneous olefin catalyst.

In certain embodiments, the support material can be silica. Silica is a common support material because it is thermally and mechanically stable and provides a relatively large number of silanol functional groups, which allow for the bonding of various organic molecules. In addition, silica also typically lacks strongly Lewis-acidic properties that could influence organic reactions. In certain embodiments, mesoporous micelle template silica (MTS) of the M41S family can be utilized as the catalyst support material. Mesoporous silicas typically exhibit a narrow pore width distribution and have a very high surface, typically at least 100 m²/g. One exemplary commercially available mesoporous silica is MCM-41, which is a structurally highly ordered mesoporous silica material having a well-defined pore width, for example, having pores ranging between 2-15 nm, and a high surface area, for example between 100 and 1500 m²/g. In certain embodiments, the surface area is between 100 and 400 m²/g, alternatively between 250 and 750 m²/g, or alternatively between 500 and 1500 m²/g. One advantage to using MCM-41 as a support material is that even when organic groups are grafted onto the inner surface of the material, the pores are still wide enough to allow for the diffusion of both the reactant and products therethrough.

In one embodiment, the catalyst is a ruthenium based homogeneous catalyst. In alternate embodiments, the catalyst is a ruthenium carbene complex. In yet another embodiment, the catalyst is a ruthenium carbene Grubbs catalyst. In certain other embodiments, the catalyst is a first generation Grubbs catalyst. In alternate embodiments, the catalyst is a second generation Grubbs catalyst.

In one embodiment, the hybrid catalyst includes a silica support material and a first generation Grubbs catalyst. In another embodiment, the hybrid catalyst includes a titania support and a first generation Grubbs catalyst. In another embodiment, the hybrid catalyst includes a silica support and a second generation Grubbs catalyst. In yet another embodiment, the hybrid catalyst includes a titania support and a second generation Grubbs catalyst. In alternate embodiments, the catalyst can be selected from a first and second generation Grubbs catalyst, and the support can be alumina.

In one embodiment, the catalyst is a molybdenum based homogeneous catalyst. In an alternate embodiment, the catalyst is a molybdenum carbene complex. In yet other embodiments, the catalyst is a molybdenum carbene Schrock catalyst.

In certain embodiments, wherein the support material of the hybrid catalyst is silica or alumina, the surface of the support material can be pre-treated prepare a sufficient quantity of hydroxyl groups on the surface of the support material. The catalyst material, preferably a Grubbs catalyst, having a chloride ligand, is contacted with the support material, in the presence of hydroxyl groups of the support material, to graft the catalyst to the surface of the support material.

The hybrid catalysts described in the present invention have the advantage of easy separation of the catalyst from the product stream, while also exhibiting higher selectivity toward the production of primary products, the ability to operate at relatively high efficiency at lower reaction temperatures, and better overall control of the reaction.

### Methods for Preparing the Hybrid Catalyst

In another aspect, a hybrid catalyst can be prepared by immobilizing a homogeneous catalyst, such as tungsten, molybdenum, and rhenium chlorides or carbonyls, on the silica support. The resulting hybrid catalyst can then be washed, dried and utilized in the metathesis reactions.

In another aspect, the present invention is directed to methods for the immobilization of olefin metathesis catalysts onto the surface of a solid support materials. In one embodiment, the surface of the support material can be functionalized with a ligand, hereinafter designated as L'. The ligands can be halogen groups, such as fluoro, chloro, bromo, and iodo groups, that may be attached to the surface through ligand exchange or by reaction with hydroxyl groups present on the surface of the support material. The functionalization of the surface of the support material can be accomplished either by grafting ligand L' to the surface, or by incorporation of the ligand onto the surface of the support material using a co-condensation method. In certain embodiments, it is preferred to graft the ligand to the surface of the support material. When using a co-condensation method for the preparation of the catalyst, the ligand can be attached to the surface of the support material, and then in a second subsequent step, the catalyst can be attached to the ligand. Subsequently, an olefin metathesis catalyst (MLn) can be introduced and bonded to the surface of the support material via a ligand exchange reaction. Until the present invention, methods for anchoring a homogeneous olefin catalyst, such as the Grubbs and Schrock catalysts, to a solid support had not been reported. Specifically, the use of surface functional groups, ligands, exchange methods, and the like, were unknown. Exemplary ligands for the functionalization of the support material surface include, hydroxyl groups, sulfonic groups, sulfonate groups, and the like.

In a embodiment of a method for preparing the hybrid catalyst of the present invention, an organometallic complex (homogeneous olefin catalyst) that includes a desired metal-functionalized ligand ratio is prepared according to known methods. After preparation, the organometallic complex can then be chemically grafted onto the support material surface by chemical reaction, such as by the silylation of a silica support material, for example by using chlorosilanes, alkoxysilanes, disilylaznes, or the like, in which a hydroxyl group present on the surface of the silica support material can be utilized as the tethering site.

During the preparation of the hybrid catalysts, the amount of catalyst that can be loaded onto the solid support will depend upon the number of hydroxyl groups on the surface of the solid support that are available for attachment. For example, the density of hydroxyl groups on the support material can range from 4 to 45 OH⁻ groups/nm², alternatively from 4 to 25 OH⁻ groups/nm², or alternatively from 25 to 45 OH⁻ groups/nm².. For example, a typical silica gel has a pore diameter of between 2.2 and 2.6 nm, although the pore size may range in certain embodiments from between 2 and 3 nm. The silica gel can have a surface area of between 750 to 800 m²/g, although in certain embodiments the surface area may range between 700 and 850 m²/g or even between 600 and 900 m²/g. Apparent bulk density of the silica gel can range between 0.67 and 0.75 g/cm³, alternatively between 0.6 and 0.8 g/cm³. Physical properties of alumina, titania, and hafnia are similar to those found for silica.

### Use of the Hybrid Catalyst

The catalysts described herein can be used in a variety of reactions, including the following exemplary reactions, for the cross-metathesis and self-metathesis of butenes. In certain embodiments, the catalyst can be utilized in a fix bed reactor operating at LHSV of between about 0.5 and 4 hr⁻¹.

CH₂=CH₂ + CH₂=CH-CH₂-CH₃ → 2 CH₃-CH=CH₂

2 CH₂=CH-CH₂-CH₃ → CH₂=CH₂ + CH₃-CH₂-CH=CH-CH₂-CH₃

CH₂=CH-CH₂-CH₃ + CH₃-CH=CH-CH₃ → CH₂=CH-CH₃ + CH₃-CH=CH-CH₂-CH₃

The hybrid catalyst of the present invention can be used for metathesis reactions between two olefin molecules. More specifically, the present invention is directed to olefin metathesis reactions to make high value propylenes from either the cross metathesis of ethylene and 2-butene, or from the self-metathesis of butenes such as 1-butene and 2-butene to make propylene and 2-pentene, or to make ethylene and 3-hexene.

In certain embodiments, the catalysts of the present invention, however, can reduce or eliminate many of the problems associated with operating olefin metathesis processes at high temperatures, such as the unwanted isomerization of olefin molecules either in the feed or product, and improving the selectivity of the products. In certain embodiments, use of the hybrid catalysts of the present invention at reaction temperatures of less than 100°C reduces unwanted isomerization products by at least 5%, alternatively at least 10%, alternatively at least 15%, alternatively at least 20%, as compared with the use of commercial hetereogeneous catalysts, such as tungsten oxide/silica, at temperatures greater than 250°C.

In certain embodiments of the present invention, the hybrid catalysts herein produce less coke than prior art olefin metathesis catalysts, thereby decreasing or eliminating the need for frequent regeneration of catalyst. In certain embodiments, the present hybrid catalysts operating at less than 100°C, produce 10% less coke than a homogeneous catalyst, such as tungsten oxide/silica, operating at a temperature of at least 250°C. Alternatively, the hybrid catalyst produces 25% less coke, alternatively 40% less coke, alternatively 50% less coke, than a heterogeneous catalyst, such as tungsten oxide/silica, operating at a temperature of at least 250°C.

In one aspect, the present invention can reduce the operating temperature for the production of propylene from ethylene and butenes by olefin metathesis. In certain embodiments utilizing the hybrid catalysts described herein, the operation temperature can be reduced by at least 100°C, alternatively between 150 and 250°C, or alternatively at least 250°C, as compared with utilizing commercial heterogeneous catalysts, such as tungsten oxide/silica. In certain embodiments, the operation temperature using the hybrid catalyst described herein will be between 100 and 200°C, alternatively between 200 and 300°C, alternatively between 100 and 300°C. By decreasing the operating temperature of the olefin metathesis process, the hybrid catalysts of the present invention can reduce overall operation energy costs. In certain embodiments, use of the catalysts described herein reduces the rate in which the catalyst is deactivated by up to 50%.

In another aspect, the present invention is directed to the use of the hybrid catalysts for olefin metathesis reactions having improved reactor temperature control, higher selectivity, and increased catalyst life.

In general, olefin metathesis reactions are preferably conducted at lower temperatures, Without wishing to be bound by any single theory, it is believed that the Bronsted or Lewis acid sites on the surface of catalyst support material have a higher acidity at the higher reaction temperatures than they do at the lower reaction temperature. A lower acidity of the support material reduces the catalytic activity of the support material, thereby reducing the likelihood for undesired isomerization of olefin molecules, either in the feed or product. Thus, by eliminating or reducing unwanted isomerization reactions, the hybrid catalyst of the present invention can improve product selectivity.

Homogeneous catalysts are generally not desirable for hydrocarbon refining and petrochemical industries. Homogeneous catalysts are typically mixed into the same liquid phase as the products and reactants, and separation of the homogeneous catalyst from the products and/or reactants can be both difficult and not economically feasible. Because of the difficulties in separating the homogeneous catalysts, Grubbs catalysts have not been previously used in refining and petrochemical applications, such as the cross metathesis of alkenes.

One advantage of the hybrid catalyst, in addition to ease of separation from products and reactants of the olefin metathesis reaction, is improved control of the reaction at much lower temperatures. The hybrid catalysts described herein operate effectively at temperatures as low as 50°C, whereas the commercially available WO₃/SiO₂ catalyst requires operating temperatures of at least 350°C, preferably at least 400°C.

### Example 1

In a first example, a Grubbs first generation catalyst can be loaded onto a silica support material as follows:

≡ SiOH + Cl-C₄₃H₇₂ClP₂Ru → ≡SiO-C₄₃H₇₂ClP₂Ru + HCl

In certain embodiments, it is assumed that only about 1% of the hydroxyl groups present on the surface of the support material will react with the Grubbs catalyst to produce the hybrid catalyst of the present invention. In the present example, approximately 0.05 g of the first generation Grubbs catalyst (benzylidene-bis(tricyclohexylphosphine)dichlororuthenium) is dissolved in approximately 10 mL of toluene. The catalyst is then loaded onto the silica gel support by impregnation with the incipient wetness method. In general, the Grubbs catalyst in the toluene solution is contacted with the silica gel for at least about 30 min., alternatively between about 10 and 20 min. The resulting silica gel, having been impregnated with the Grubbs catalyst, is removed from the toluene solution and dried in a vacuum oven at about 50°C for at least 2 hours, alternatively at least 4 hours. The resulting dried, impregnated catalyst support material is then contacted with the toluene solution containing the Grubbs catalyst for sufficient time to further impregnate the support material, and dried under vacuum. The steps of contacting the support material with the toluene solution can be repeated until no toluene solution remains. The procedure can be used for either first or second generation Grubbs catalysts.

The above described incipient wetness method can be used to impregnate a solid silica support material with a catalyst mixture. For example, in certain embodiments, the mixture can include various ruthenium carbene catalysts, such as a mixture that includes Grubbs first and second generation ruthenium carbene catalysts, wherein the first generation Grubbs catalyst has a molecular formula C₄₃H₇₂Cl₂P₂Ru (IUPAC name benzylidene-bis(tricyclohexylphosphine)dichlororuthenium) and the second generation Grubbs catalyst has a molecular formula C₄₆H₆₅Cl₂N₂PRu (IUPAC name benzylidene[1,3- bis(2,4,6-trimethylphenyl)-2- imidazolidinylidene]dichloro(tricyclohexylphosphine)ruthenium). In certain embodiments, the Hoveyda-Grubbs catalyst can also be used to prepare the hybrid catalyst.

### Example 2

A comparison of the activity, selectivity, and reaction conditions for a commercially available heterogeneous catalyst (WO₃/SiO₂), an unsupported homogeneous catalyst (first generation Grubbs catalyst; benzylidene-bis(tricyclohexylphosphine)dichlororuthenium), and the hybrid catalyst of the present invention (first generation Grubbs catalyst on SiO₂ support) is provided in Table 1, below. The olefin metathesis reaction conditions for each catalyst are listed, and as shown, the commercial heterogeneous is shown to have higher conversion, lower selectivity and requires substantially greater operating temperatures. The olefin feed for the metathesis reaction is a mixture of 1-butene and 2-butene ranging from a 40:60 to a 50:50 mixture thereof.

**Table 1.**

| | Commercial Heterogeneous Catalyst | Commercial Heterogeneous Catalyst | Unsupported Homogeneous Catalyst | Hybrid Catalyst (homogeneous supported catalyst) |
|---|---|---|---|---|
| Conversion | 60% | 65.7% | 53.3% | ∼ 53.5% |
| Selectivity | 27% | 45.8% | 49% | ∼ 49% |
| Reaction Temperature | 350°C | 400°C | 50°C | ∼ 50°C |
| Reaction Pressure | 20 bar | 20 bar | 20 bar | ∼ 20 bar |
| C7+ products | 2.5% | 4.2% | ∼ 0% | ∼ 0% |

With an olefin feed that is pure 1-butene, the hybrid catalyst can have a selectivity of at least 95%, alternatively at least 97%, or alternatively at least 99%. In comparison, commercial WO₃/SiO₂ system are typically a lot less selective with considerable amounts of propene and 2-pentene being produced, due to a high isomerization activity.

Although the present invention has been described in detail, it should be understood that various changes, substitutions, and alterations to the catalysts, methods for preparing the catalysts, and use of the catalysts can be made without departing from the principle and scope of the invention. Accordingly, the scope of the present invention should be determined by the following claims and their appropriate legal equivalents.

The singular forms "a", "an" and "the" include plural referents, unless the context clearly dictates otherwise.

Optional or optionally means that the subsequently described event or circumstances may or may not occur. The description includes instances where the event or circumstance occurs and instances where it does not occur.

Ranges may be expressed herein as from about one particular value, and/or to about another particular value. When such a range is expressed, it is to be understood that another embodiment is from the one particular value and/or to the other particular value, along with all combinations within said range.

Throughout this application, where patents or publications are referenced, the disclosures of these references in their entireties are intended to be incorporated by reference into this application, in order to more fully describe the state of the art to which the invention pertains, except when these references contradict the statements made herein.

Embodiments of the invention numbered 1 to 19 are provided below.
1. A hybrid olefin hydration catalyst, comprising:
   a homogeneous olefin metathesis catalyst appended to a solid support material;
   wherein the olefin metathesis catalyst is selected from a homogeneous tungsten-based, molybdenum-based, or ruthenium-based catalyst;
   wherein the solid support is a high surface area porous support; and
   wherein the catalyst is attached to the solid support with a ligand.
2. The catalyst of embodiment 1, wherein the solid support is selected from silica, alumina, titania, zirconia, hafnia, and niobia.
3. The catalyst of embodiment 1, wherein the solid support is activated carbon.
4. The catalyst of embodiment 1, wherein the solid support is a surface modified ion exchange cross-linked polystyrene resin, wherein the surface of the support is modified with sulfonic or sulfonate groups.
5. The catalyst of embodiment 1, wherein the olefin metathesis catalyst is benzylidene-bis(tricyclohexylphosphine)dichlororuthenium.
6. The catalyst of embodiment 1, wherein the olefin metathesis catalyst is a benzylidene[1,3- bis(2,4,6-trimethylphenyl)-2-imidazolidinylidene] dichloro (tricyclohexyl phosphine) ruthenium.
7. The catalyst of embodiment 1, wherein the olefin metathesis catalyst is selected from molybdenum hexacarbonyl (Mo(CO)₆), rhenium pentacarbonyl (Re(CO)₅), ruthenium tetracarbonyl (Ru(CO)₄), (methylmethoxycarbene) pentacarbonyl tungsten (CO)₅W=C(CH₃)(OCH₃), tungsten hexachloride, methyllithium or tetramethyltin and molybdenum pentachloride.
8. A method of preparing the a hybrid olefin metathesis catalyst; the method comprising the steps of:
   providing a surface modified solid support material, the solid support material including a ligand capable of participating in a ligand exchange reaction;
   appending a homogeneous olefin catalyst to a solid support material via ligand exchange reaction, wherein the ligand is selected from a fluorine, chlorine, bromine, or iodine ion.
   recovering the hybrid olefin metathesis catalyst.
9. The method of embodiment 8, wherein the solid support is selected from silica, alumina, titania, zirconia, hafnia, and niobia.
10. The method of embodiment 8, wherein the solid support is activated carbon.
11. The method of embodiment 8, wherein the solid support is a polymer selected from the group consisting of an ion exchange cross-linked resin.
12. The method of embodiment 9, wherein the homogeneous olefin catalyst is selected from benzylidene-bis(tricyclohexylphosphine)dichlororuthenium and benzylidene[1,3-bis(2,4,6-trimethylphenyl)-2-imidazolidinylidene]dichloro(tricyclohexyl phosphine)ruthenium.
13. A method of preparing the hybrid olefin metathesis catalyst of embodiment 1; the method comprising the steps of:
   providing a surface modified high surface area solid support material, the solid support material including hydroxyl groups appended thereto; and
   contacting the surface modified solid support material with a homogeneous olefin metathesis catalyst comprising a metal selected from the group consisting of tungsten, molybdenum and
   ruthenium, such that the catalyst binds to the surface of the support material to produce the hybrid catalyst.
14. The method of embodiment 13, wherein the step of contacting the surface modified solid support material with a homogeneous olefin metathesis catalyst comprises dissolving the homogeneous olefin metathesis catalyst in an organic solvent and contacting the catalyst containing organic solvent
15. The method of embodiment 13, wherein the homogeneous olefin catalyst is selected from benzylidene-bis(tricyclohexylphosphine)dichlororuthenium and benzylidene[1,3- bis(2,4,6-trimethylphenyl)-2- imidazolidinylidene]dichloro(tricyclohexyl phosphine)ruthenium.
16. The method of embodiment 13, wherein the solid support is selected from silica, alumina, titania, zirconia, hafnia, and niobia.
17. A method for the metathesis of butene to produce propene, the method comprising:
   providing an olefin feedstream comprising 1-butene, 2-butene or a mixture thereof to a reaction chamber;
   contacting the olefin feedstream with the heterogeneous olefin metathesis catalyst of claim 1 at a temperatures of less than about 100°C, said catalyst comprising a ruthenium-based, molybdenum-based, or tungsten based olefin metathesis catalyst appended to a solid support;
      withdrawing a product stream comprising propene; and
   separating the catalyst from the product stream.
18. The method of embodiment 17, wherein the catalyst includes a ruthenium-based catalyst selected from benzylidene-bis(tricyclohexylphosphine)dichlororuthenium and benzylidene[1,3- bis(2,4,6-trimethylphenyl)-2- imidazolidinylidene]dichloro(tricyclohexyl phosphine)ruthenium.
19. The method of embodiment 17, wherein the solid support is selected from silica, alumina, titania, zirconia, hafnia, and niobia.

## Claims

1. A method of preparing a hybrid olefin metathesis catalyst, the method comprising the steps of:
contacting an olefin metathesis catalyst, optionally dissolved in an organic solvent, with a silica support that includes a halogen or hydroxyl ligand capable of participating in a ligand exchange reaction;
appending the olefin metathesis catalyst to the silica support via the ligand exchange reaction to form a hybrid olefin metathesis catalyst; and
recovering the hybrid olefin metathesis catalyst.

2. The method of claim 1, wherein the silica support is mesoporous silica.

3. The method of any preceding claim, wherein the olefin metathesis catalyst comprises a metal selected from the group consisting of tungsten, molybdenum and ruthenium.

4. The method of any preceding claim, wherein the olefin metathesis catalyst is benzylidene-bis(tricyclohexylphosphine)dichloro ruthenium and/or benzylidene[1,3-bis(2,4,6-trimethylphenyl)-2-imidazolidinylidene]dichloro(tricyclohexylphosphine) ruthenium.

5. The method of claim 4, where the organic solvent is toluene.

6. The method of claim 5, the step of contacting the olefin metathesis catalyst with the silica support is performed by an incipient wetness method.

7. The method of any preceding claim, wherein the ligand is hydroxyl.

8. The method of any preceding claim, in which the density of hydroxyl ligands on the surface of the silica support that are available for attachment in the contacting step is from 4 to 45 OH groups / (nm)².

9. The method of any of claims 1 to 6, where the ligand is a halogen ligand.

10. The method of any preceding claim, where the halogen ligand is selected from the group consisting of fluoro, chloro and iodo groups.

11. The method of any preceding claim in which the silica support is a silica gel with a pore size from between 2 and 3 nm.

12. The method of any preceding claim in which the silica support is a silica gel with a bulk density between 0.6 and 0.8 g / cm³.

13. The method of any preceding claim, further comprising washing and drying the hybrid olefin metathesis catalyst obtained from the recovery, optionally wherein the resulting washed and dried hybrid olefin metathesis catalyst is used in a metathesis reaction.

14. The method of claim 2, wherein the mesoporous silica is micelle template silica (MTS) of the M41S family.

15. The method of claim 2, wherein the mesoporous silica is MCM-41 having a pore width ranging between 2 to 15 nm, and a surface area between 100 and 1500 m²/g.
